# EUROPEAN PATENT APPLICATION

(11) **EP 4 835 509 A1**
(43) Date of publication of application: **04.11.2026**
(21) Application number: 26176078.9
(22) Date of filing: 01.05.2026
(51) Int. Cl.: G01R 33/563, G01R 33/483

(54) **MRI APPARATUS**

(30) Priority: 02.05.2025 US 202519196847
(71) Applicant: The Regents of the University of California, Oakland, CA 94607-5200 (US); Canon Kabushiki Kaisha, Tokyo 146-8501 (JP)
(72) Inventor: MIYAZAKI, Mitsue, Oakland, 94607-5200 (US); MALIS, Vadim, Oakland, 94607-5200 (US); KASSAI, Yoshimori, Tokyo, 146-8501 (JP)
(74) Representative: Marks & Clerk LLP

(57) **Abstract**

In one embodiment, an MRI apparatus includes a setting unit configured to set a pulse sequence group to be applied to a tissue or organ into which body fluid flows from a plurality of inflow paths, the pulse sequence group being provided with RF pulses for labeling body fluid in the plurality of inflow paths; a generation unit configured to generate perfusion data regarding perfusion of the body fluid flowing into the tissue or organ of the body fluid from each of the plurality of inflow paths based on MR signals that are emitted from an object by application of the pulse sequence group and received with a receiving coil; and a calculation unit configured to calculate metrics related to the perfusion in the tissue or organ for each of the plurality of inflow paths based on the perfusion data.

## Description

### FIELD

Disclosed embodiments relate to a magnetic resonance imaging (MRI) apparatus.

### BACKGROUND

An MRI apparatus is an imaging apparatus that excites nuclear spin of an object placed in a static magnetic field by applying a radio frequency (RF) signal having the Larmor frequency and reconstructs an image on the basis of magnetic resonance (MR) signals emitted from the object due to the excitation.

Among the imaging methods using an MRI apparatus, an imaging method called perfusion imaging is known. The perfusion imaging using an MRI apparatus includes both non-contrast imaging without using a contrast agent and dynamic contrast-enhanced imaging (DCE) in which gadolinium-based contrast agents (GBCA) are externally administered.

Since the DCE is an examination in which contrast agents are used, the DCE is not an examination applicable to all the patients. For example, it is said that the DCE cannot be applied to patients with a renal disease.

It is known that the liver has two blood vessels including the hepatic artery and the portal vein as blood inflow paths. When using the DCE, however, the entire liver is stained with a contrast agent, so it is not possible to distinguish which blood vessel has carried the cause of the disease.

The non-contrast imaging method is also called an Arterial Spin Labeling (ASL) method. The ASL method is an imaging method in which blood labeled by an RF signal is used in place of a contrast agent and there is no need to administer a contrast agent from outside, and thus, the ASL method is excellent in simplicity and non-invasiveness.

However, the conventional ASL method is on the premise that the number of blood flow paths into a tissue or organ is one. In order to quantify metrics obtained by perfusion imaging, it is important to model an input path (e.g., artery), the tissue or organ, and an output path (e.g., vein). In the conventional ASL method, however, models with only one input path are exclusively used, and so far, there have been no known ASL method and/or its analysis method corresponding to a model in which the number of input paths to one tissue or organ is two or more.

### SUMMARY OF THE INVENTION

One of the problems to be solved by the embodiments disclosed in the specification and drawings is that the state of the tissue or organ can be evaluated for each inflow path in more detail. It should be noted, however, that possible problems to be solved by the embodiments disclosed in the present specification and the drawings are not limited to the problem presented above. It is also possible to regard problems corresponding to the advantageous effects achieved by the configurations described in the following embodiments as other problems.

In one embodiment, an MRI apparatus includes a setting unit configured to set a pulse sequence group to be applied to a tissue or organ into which body fluid flows from a plurality of inflow paths, the pulse sequence group being provided with RF pulses for labeling body fluid in the plurality of inflow paths; a generation unit configured to generate perfusion data regarding perfusion of the body fluid flowing into the tissue or organ of the body fluid from each of the plurality of inflow paths based on MR signals that are emitted from an object by application of the pulse sequence group and received with a receiving coil; and a calculation unit configured to calculate metrics related to the perfusion in the tissue or organ for each of the plurality of inflow paths based on the perfusion data.

The inflow path may be an inflow blood vessel, and the body fluid may be blood.

The pulse sequence group to be set by the setting unit may be a pulse sequence group corresponding to an imaging method in which (a) a tag pulse is applied to a predetermined tag region including a specific inflow path among the plurality of inflow paths and then tag-on data are acquired from a predetermined imaging region in the tissue or organ with a plurality of different delay times TI from application of the tag pulse, and (b) tag-off data are acquired from the predetermined imaging region without applying the tag pulse.

The pulse sequence group to be set by the setting unit may include a first pulse sequence group for acquiring the tag-on data and a second pulse sequence group for acquiring the tag-off data, wherein the first pulse sequence group is a pulse sequence group in which a tag-on sequence is repeated for the plurality of delay times TI, the tag-on sequence including a first region non-selective IR pulse, a first region selective IR pulse as the tag pulse subsequent thereto, a first acquisition sequence provided after the delay time TI from application of the first region selective IR pulse; and the second pulse sequence group is a pulse sequence group in which a tag-off sequence including a second region non-selective IR pulse and a second acquisition sequence is repeated for the plurality of delay times TI, the tag-off sequence being a sequence in which an interval between the second region non-selective IR pulse and the second acquisition sequence is set to be same as an interval between the first region non-selective IR pulse and the first acquisition sequence.

The first pulse sequence group to be set by the setting unit may include a suppression pulse applied immediately before the first acquisition sequence, the suppression pulse being for suppressing a signal of inflow body fluid from at least one inflow path excluding the specific inflow path among the plurality of inflow paths; and the second pulse sequence group to be set by the setting unit may include the suppression pulse applied immediately before the second acquisition sequence.

The pulse sequence group to be set by the setting unit may include a first pulse sequence group for acquiring the tag-on data and a second pulse sequence group for acquiring the tag-off data; wherein the first pulse sequence group is a pulse sequence group in which a tag-on sequence is repeated for the plurality of delay times TI, the tag-on sequence including a first region non-selective IR pulse, a first region selective IR pulse as the tag pulse subsequent thereto, a first acquisition sequence provided after the delay time TI from application of the first region selective IR pulse; and the second pulse sequence group is a pulse sequence group in which a tag-off sequence including a second region non-selective IR pulse and a second acquisition sequence is repeated for the plurality of delay times TI, the tag-off sequence being a sequence in which an interval between the second region non-selective IR pulse and the second acquisition sequence is set to be same as an interval between the first region non-selective IR pulse and the first acquisition sequence.

The generation unit may be further configured to calculate a perfusion curve in the tissue or organ for each of the plurality of inflow paths based on the perfusion data.

The MRI apparatus may further include a display control unit may display at least one of the following perfusion curves; respective perfusion curves calculated for the plurality of inflow paths, and a perfusion curve obtained by combining at least two of the respective perfusion curves calculated for the plurality of inflow paths.

The generation unit may further combine at least two of the respective perfusion curves calculated for the plurality of inflow paths.

The calculation unit may be further configured to, by using the calculated perfusion curve, calculate at least one of peak height, mean transition time, time to peak, relative blood fluid volume, and relative blood flow, as the metrics related to the perfusion for each of the plurality of inflow paths.

The MRI apparatus may further include a memory configured to store an evaluation database in which standard metrics related to perfusion curves obtained by measurement on a plurality of healthy persons are accumulated, and an evaluation unit configured to evaluate a state of the tissue or organ of the object by comparing the metrics calculated from the perfusion curve of the object with the standard metrics of the evaluation database.

The tissue or organ may be a liver, and the specific inflow path may be one blood vessel selected from a group consisting of a hepatic artery, a portal vein, a splenic vein, and a superior mesenteric vein.

The generation unit may be further configured to divide entirety of the liver into a plurality of subregions and calculate, by using the perfusion data, perfusion curves corresponding to respective inflow paths of the hepatic artery, the portal vein, the splenic vein, and the superior mesenteric vein for each of the plurality of subregions, wherein the tissue or organ is a liver, the specific inflow path is one blood vessel selected from a group consisting of a hepatic artery, a portal vein, a splenic vein, and a superior mesenteric vein.

The MRI apparatus may further include an evaluation unit configured to classify the subregions depending on which of the hepatic artery, the portal vein, the splenic vein, and the superior mesenteric vein is a dominant source of inflow body fluid, by using at least one of shapes of calculated perfusion curves corresponding to respective inflow paths and metrics calculated from the perfusion curves.

The MRI apparatus may further include an evaluation unit configured to evaluate which inflow path among the hepatic artery, the portal vein, the splenic vein, and the superior mesenteric vein is a source of origin for a tumor occurring in a subregion, by using at least one of shapes of calculated perfusion curves corresponding to respective inflow paths and metrics calculated from the perfusion curves.

The tissue or organ may be a psoas muscle or a paraspinal muscle; and the specific inflow path may be a feeding vessel for the psoas muscle or the paraspinal muscle and is at least one specific segmental artery among a plurality of segmental arteries branching from a descending aorta.

The generation unit may be further configured to calculate a perfusion curve corresponding to the at least one specific segmental artery among the plurality of segmental arteries by using the perfusion data, wherein the tissue or organ is a psoas muscle or a paraspinal muscle, and the specific inflow path is a feeding vessel for the psoas muscle or the paraspinal muscle and is at least one specific segmental artery among a plurality of segmental arteries branching from a descending aorta.

The MRI apparatus may further include an evaluation unit configured to evaluate which of the plurality of segmental arteries has stopped supply of inflow blood or is reduced in supply of inflow blood, by using at least one of shapes of calculated perfusion curves corresponding to inflow blood from respective segmental arteries and metrics calculated from the perfusion curves.

The tissue or organ may be a brain; and the specific inflow path may be at least one artery of right or left internal carotid arteries, or right or left vertebral arteries.

### BRIEF DESCRIPTION OF THE DRAWINGS

Fig. 1 is a configuration diagram illustrating a configuration of an MRI apparatus according to one embodiment.
Fig. 2 is a block diagram illustrating the MRI apparatus according to the embodiment.
Fig. 3 is a flowchart illustrating operation of the MRI apparatus according to the embodiment.
Fig. 4A and Fig. 4B are schematic diagrams illustrating a pulse sequence group to be used in the MRI apparatus.
Fig. 5A to Fig. 5D are schematic diagrams illustrating a pulse sequence group to be used in the MRI apparatus.
Fig. 6 is a schematic diagram illustrating inflow blood vessels with respect to the liver.
Fig. 7 is a schematic diagram illustrating application regions of respective RF pulses such as a tag pulse for labeling blood flowing into the liver from the portal vein.
Fig. 8 is a schematic diagram illustrating a concept of processing of generating perfusion images from tag-on images and tag-off images.
Fig. 9A to Fig. 9C are schematic diagrams illustrating a concept of processing of calculating a perfusion curve and metrics related to the perfusion from perfusion images.
Fig. 10 is a schematic diagram illustrating application regions of respective RF pulses such as a tag pulse for labeling the blood flowing into the liver from the splenic vein.
Fig. 11 is a schematic diagram illustrating application regions of respective RF pulses such as a tag pulse for labeling the blood flowing into the liver from the superior mesenteric vein.
Fig. 12 is a schematic diagram illustrating application regions of respective RF pulses such as a tag pulse for labeling the blood flowing into the liver from the hepatic artery.
Fig. 13 is a graph illustrating respective perfusion curves derived from the hepatic artery, the portal vein, the splenic vein, and the superior mesenteric vein.
Fig. 14 is a conceptual diagram illustrating processing of classifying each subregion of the liver on the basis of which of the hepatic artery, the portal vein, the splenic vein, and the superior mesenteric vein is the dominant source of inflow blood.
Fig. 15A and Fig. 15B are schematic diagrams illustrating the psoas muscle and the paraspinal muscle in the human body.
Fig. 16A and Fig. 16B are conceptual diagrams illustrating processing of selecting a specific segmental artery from among a plurality of segmental arteries and calculating the perfusion curve of inflow blood from the selected segmental artery.
Fig. 17A to Fig. 17C are conceptual diagrams for illustrating processing of (a) using the calculated perfusion curves to determine presence/absence of an abnormality in blood supply from the plurality of segmental arteries and (b) determining which segmental artery is the cause of the abnormality in the case of presence of the abnormality.

### DETAILED DESCRIPTION

Hereinbelow, respective embodiments of an MRI apparatus 1 will be described by referring to the accompanying drawings.

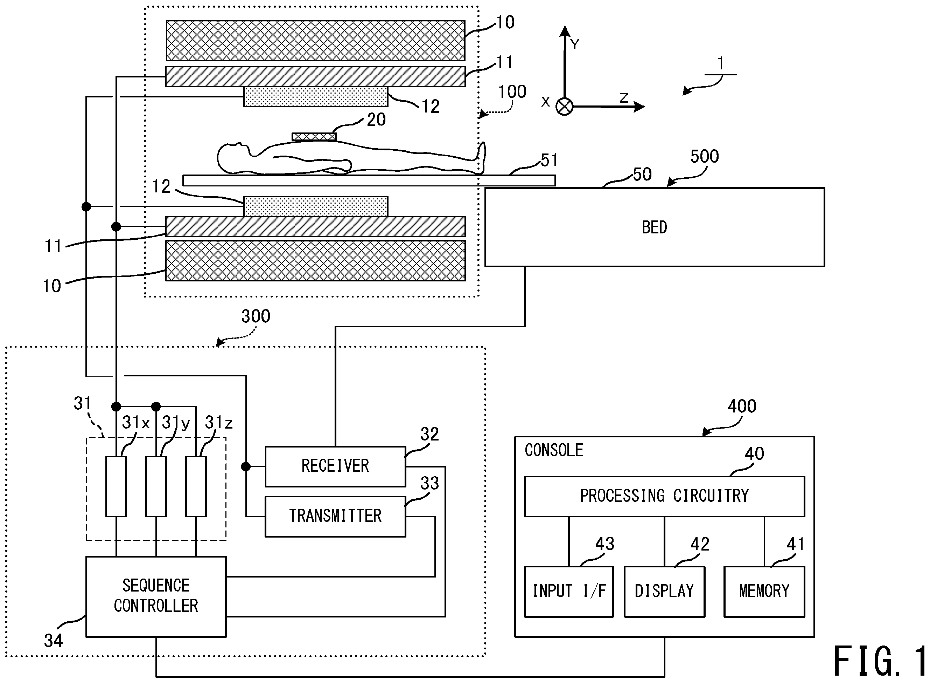

Fig. 1 is a block diagram illustrating an overall configuration of the MRI apparatus 1 according to one embodiment. The MRI apparatus 1 includes a gantry 100, a control cabinet 300, a console 400, and a bed 50, for example.

The gantry 100 includes a static magnetic field magnet 10, a gradient coil 11, and a whole body (WB) coil 12, and these components are housed in a cylindrical housing. The bed 500 includes a bed body 50 and a table 51. Further, the MRI apparatus 1 includes an array coil 20 to be disposed close to an object.

The control cabinet 300 includes three gradient-coil power supplies 31 (31x for the X-axis, 31y for the Y-axis, and 31z for the Z-axis), a receiver 32, a transmitter 33, and a sequence controller 34.

The static magnetic field magnet 10 of the gantry 100 is substantially in the form of a cylinder, and generates a static magnetic field inside a bore, which is an imaging space of an object such as a patient (i.e., space inside the cylindrical structure of the gantry 100). The static magnetic field magnet 10 includes a superconducting coil inside, and the superconducting coil is cooled down to an extremely low temperature by liquid helium. The static magnetic field magnet 10 generates a static magnetic field by supplying the superconducting coil with an electric current provided from a static-magnetic-field power supply (not shown) in an excitation mode. Afterward, the static magnetic field magnet 10 shifts to a persistent current mode, and the static-magnetic-field power supply is disconnected. Once it enters the persistent current mode, the static magnetic field magnet 10 continues to generate a strong static magnetic field for a long time, for example, over one year. Note that the static magnetic field magnet 10 may be configured as a permanent magnet.

The gradient coil 11 is also substantially in the form of a cylinder and is fixed to the inside of the static magnetic field magnet 10. This gradient coil 11 applies gradient magnetic fields to the object in the respective directions of the X-axis, the Y-axis, and the Z-axis by being supplied with electric currents from the respective gradient-coil power supplies 31x, 31y, and 31z.

The bed body 50 of the bed 500 can move the table 51 in the vertical direction and in the horizontal direction, and moves the table 51 with the object placed thereon to a predetermined height before imaging. Afterward, when the object is imaged, the bed body 50 moves the table 51 in the horizontal direction so as to move the object to the inside of the bore.

The WB body coil 12 is shaped substantially in the form of a cylinder so as to surround the object, and is fixed to the inside of the gradient coil 11. The WB coil 12 applies RF pulses transmitted from the transmitter 33 to the object, and receives MR signals emitted from the object due to excitation of hydrogen nuclei.

The array coil 20 is an RF coil that receives MR signals emitted from the object at a position close to the object. The array coil 20 is composed of a plurality of coil elements, for example. There are various models for the array coil 20 depending on an anatomical imaging part of the object, such as for the head, for the chest, for the spine, for the legs, and for the whole body. Fig. 1 illustrates an array coil 20 for the chest.

The transmitter 33 transmits an RF pulse to the WB coil 12 on the basis of an instruction from the sequence controller 34.

The receiver 32 detects the MR signals received by the WB coil 12 and/or the array coil 20, and transmits raw data obtained by digitizing the detected MR signals to the sequence controller 34.

Under the control of the console 400, the sequence controller 34 performs a scan of the object by driving the gradient-coil power supplies 31, the transmitter 33, and the receiver 32. Further, the sequence controller 34 receives the raw data from the receiver 32 by performing the scan, and transmits the raw data to the console 400.

The sequence controller 34 includes processing circuitry (not shown). This processing circuitry is composed of hardware such as a processor configured to execute predetermined programs, a field programmable gate array (FPGA), and an application specific integrated circuit (ASIC).

The console 400 is configured as a computer that includes processing circuitry 40, a memory 41, a display 42, and an input I/F 43.

The memory 41 is a recording medium including a read-only memory (ROM) and a random access memory (RAM) in addition to an external memory device such as a hard disk drive (HDD) and an optical disc device. The memory 41 stores various programs to be executed by the processor of the processing circuitry 40 as well as various data and information.

The input I/F 43 includes various devices for a user to input various data and information, such as a mouse, a keyboard, a trackball, and a touch panel.

The display 42 is a display device such as a liquid crystal display panel, a plasma display panel, and an organic EL panel.

The processing circuitry 40 is, for example, a circuit provided with a central processing unit (CPU) and/or a special-purpose or general-purpose processor. The processor implements various functions described below by executing the programs stored in the memory 41. The processing circuitry 40 may be configured as hardware such as an FPGA and an ASIC. The various functions described below can also be implemented by such hardware. Alternatively, the processing circuitry 40 can implement the various functions by combining hardware processing and software processing based on its processor and programs.

The console 400 controls the entirety of the MRI apparatus 1 by using these components. Specifically, the console 400 receives imaging conditions and other various instructions and information entered by a user such as a medical imaging technologist via the input I/F 43 including a mouse and a keyboard. The processing circuitry 40 causes the sequence controller 34 to execute a scan on the basis of the inputted imaging conditions, and reconstructs an image on the basis of the raw data transmitted from the sequence controller 34. The reconstructed image is displayed on the display 42 or stored in the memory 41.

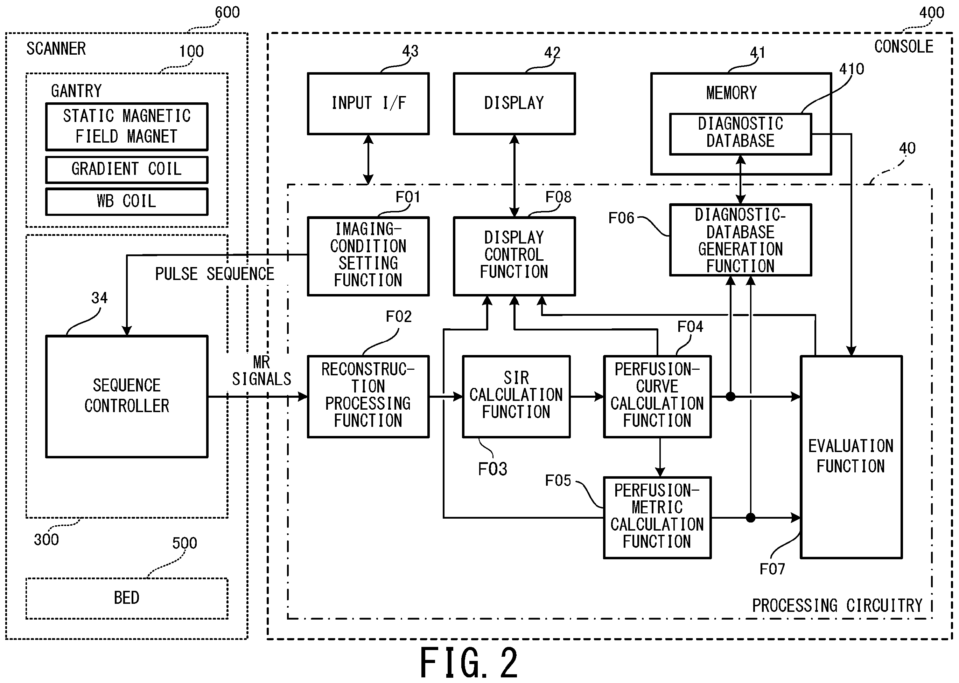

Fig. 2 is a block diagram illustrating the MRI apparatus 1 according to the embodiment, particularly focusing on the functions implemented by the processing circuitry 40 of the console 400. Among the components of the MRI apparatus 1 shown in Fig. 1, the components excluding the console 400, i.e., the control cabinet 300, the gantry 100, and the bed 500, are collectively referred to as a scanner 600 in Fig. 2.

The processing circuitry 40 implements each of an imaging-condition setting function F01, a reconstruction processing function F02, an SIR calculation function F03, a perfusion-curve calculation function F04, a perfusion-metric calculation function F05, a diagnostic-database generation function F06, an evaluation function F07, and a display control function F08.

The imaging-condition setting function F01 sets a pulse sequence group to be applied to a tissue or organ into which blood flows from a plurality of inflow blood vessels, and the pulse sequence group includes an RF pulse for labeling the blood in the plurality of inflow blood vessels. The pulse sequence group set by the imaging-condition setting function F01 has a plurality of tag-on sequences different in delay time TI from each other and a plurality of tag-off sequences different in delay time TI from each other. Specific cases of the pulse sequence group will be described below.

The three functions of the reconstruction processing function F02, the SIR calculation function F03, and the perfusion-curve calculation function F04 generate perfusion data regarding perfusion of blood flowing in from each of the plurality of inflow blood vessels in the tissue or organ for each inflow blood vessel, based on MR signals emitted from the object due to application of the above-described pulse sequence group.

Among the three functions F02, F03, and F04, the reconstruction processing function F02 generates tag-on images from tag-on data acquired by the tag-on sequences for each delay time TI, and generates tag-off images from tag-off data acquired by the tag-off sequences for each delay time TI.

The SIR calculation function F03 calculates a signal intensity ratio (SIR) as perfusion data from pixel values at the same pixel position in each of the tag-on image and the tag-off image, and generates a perfusion image in which the SIR is arranged for each delay time TI.

The perfusion-curve calculation function F04 uses the signal intensity ratio to calculate the perfusion curve in the tissue or organ for each of the plurality of inflow blood vessels for each pixel or at a specified pixel position.

The perfusion-curve calculation function F04 may calculate a combined perfusion curve by combining at least two (for example, all) of the perfusion curves calculated for the respective inflow vessels.

The perfusion-metric calculation function F05 calculates metrics related to the perfusion (hereinafter also referred to as perfusion-related metrics as appropriate), such as PH (peak height) and MTT (mean transition time), on the basis of each calculated perfusion curve.

The diagnostic-database generation function F06 accumulates the perfusion-related metrics, which are calculated from perfusion curves measured for a plurality of healthy persons, as standard metrics so as to generate a diagnostic database 410. The generated diagnostic database 410 is stored in the memory 41, and the diagnostic database 410 is updated as necessary.

The evaluation function F07, in an examination or diagnosis of an object such as a patient, compares the metrics related to the perfusion calculated from the generated perfusion curve with the standard metrics in the diagnostic database 410, and evaluates the state of the tissue or organ of the object. The evaluation function F07 may evaluate the state of the tissue or organ of the object on the basis of the perfusion curve for each inflow blood vessel or may evaluate the state of the tissue or organ of the object on the basis of the combined perfusion curve. The evaluation function F07 may evaluate the state of the tissue or organ of the object by comparing at least two of the following: the respective perfusion curves for the inflow blood vessels; and the combined perfusion curve, i.e., by comparing a plurality of perfusion curves with each other.

The display control function F08 controls the display 42 in such a manner that the display 42 displays respective perfusion curves calculated for the plurality of inflow blood vessels, at least one perfusion-related metric, evaluation results acquired by the evaluation function F07, at least one perfusion image, and at least one MR image. The display control function F08 may display at least one of the above-described items on the basis of a user selection, for example. The display control function F08 may separate the plurality of inflow blood vessels so as to display the perfusion curve for each inflow blood vessel or may display the combined perfusion curve. The display control function F08 may cause at least two of the respective perfusion curves of the inflow vessels and one combined perfusion curve to be superimposed and displayed in association with the delay time TI.

The display control function F08 may display perfusion images and perfusion curves in such a manner that the perfusion images are associated with the perfusion curves. In perfusion imaging, the perfusion-related metrics can be visualized by color coding using at least one chromatic color and/or at least on achromatic color. The display control function F08 may visualize and display perfusion-related metrics on the basis of a specific perfusion curve. For example, a color map based on TTP (time to peak) of one of the separated perfusion curves may be displayed. Additionally or alternatively, a color map based on TTP of the combined perfusion curve may be displayed.

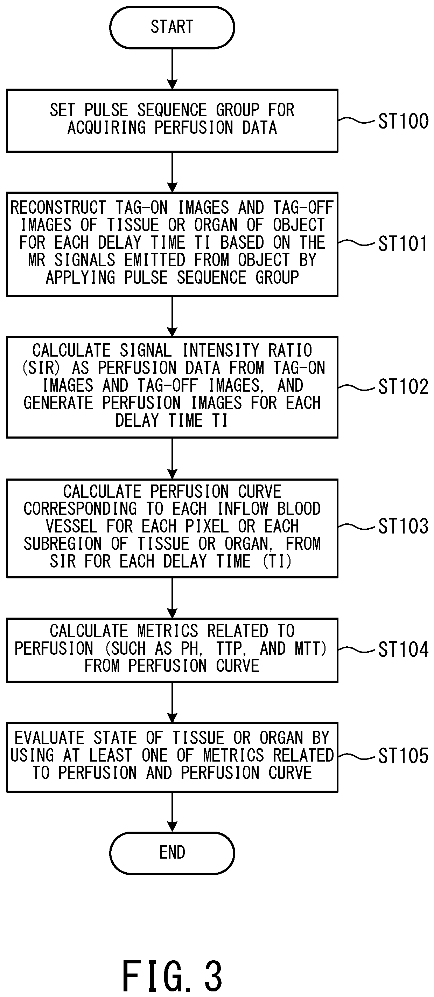

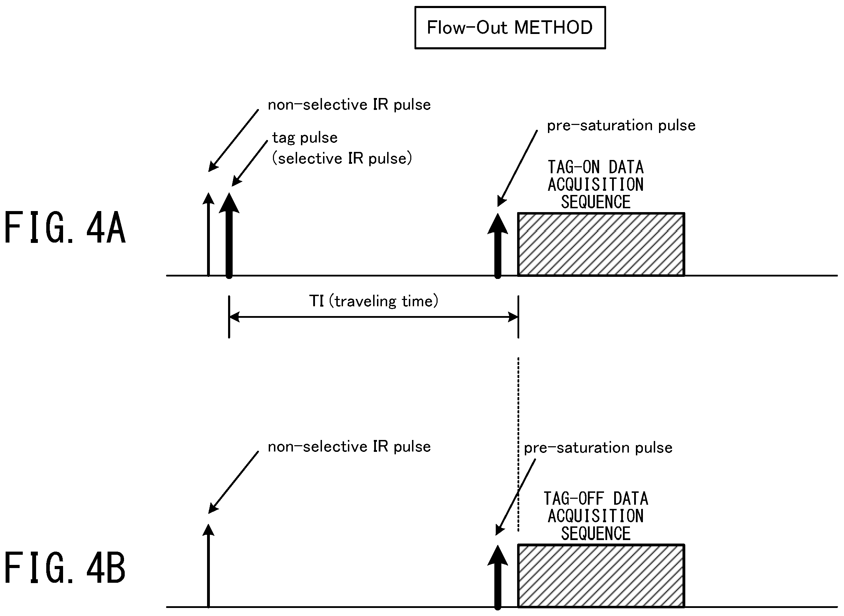

Fig. 3 is a flowchart illustrating operation of the MRI apparatus 1 according to the embodiment. Hereinbelow, the operation of the MRI apparatus 1 will be specifically described in order on the basis of the flowchart of Fig. 3 and supplementary diagrams of Fig. 4A to Fig. 17C.

In the first step ST100, a pulse sequence group for acquiring perfusion data is set. Fig. 4A to Fig. 5D illustrate the pulse sequence group to be used in the MRI apparatus 1 of the embodiment.

The pulse sequence group corresponds to an imaging method, i.e., a so-called ASL method, in which (a) a tag pulse is applied to a predetermined tag region in a specific inflow blood vessel among a plurality of inflow blood vessels and then tag-on data are acquired from a predetermined imaging region in a tissue or organ with a plurality of different delay times TI from the application of the tag pulse and (b) tag-off data are acquired from the predetermined imaging region without applying the tag pulse.

Specifically, the pulse sequence group includes a first pulse sequence group for acquiring the tag-on data and a second pulse sequence group for acquiring the tag-off data.

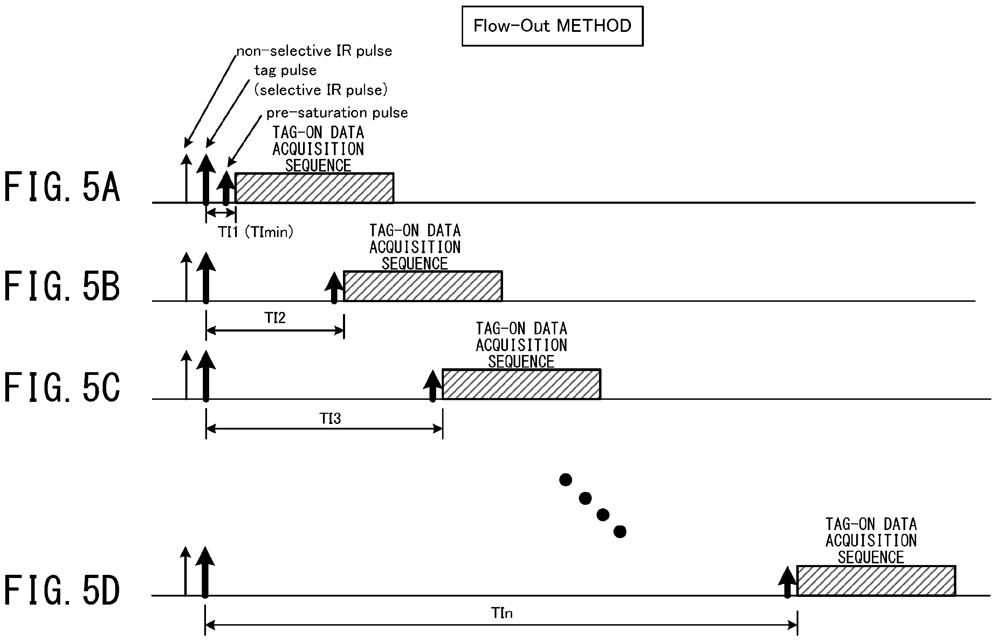

Fig. 4A to Fig. 5D illustrate the first pulse sequence group. As shown in Fig. 4A, the first pulse sequence group has a tag-on sequence that includes a first region non-selective IR pulse, a first region selective IR pulse as the tag pulse, and a first acquisition sequence (i.e., tag-on data acquisition sequence) provided after elapse of a delay time TI from application of the first region selective IR pulse. In the first pulse sequence group as shown in Fig. 5A to Fig. 5D, the tag-on sequence is repeated for the plurality of delay times TI from the shortest delay time TI1 (TI(min)) to the longest delay time TIn.

As shown in Fig. 4B, the second pulse sequence group has a tag-off sequence that includes a second region non-selective IR pulse and a second acquisition sequence (i.e., tag-off data acquisition sequence. In the tag-off sequence, the interval between the second region non-selective IR pulse and the second acquisition sequence is set to be the same as the interval between the first region non-selective IR pulse and the first acquisition sequence. In the second pulse sequence group, the tag-off sequence is repeated for the plurality of delay times TI, similarly to the first pulse sequence group.

In each of the tag-on sequences of the first pulse sequence group as shown in Fig. 4A, a suppression pulse (i.e., a pre-saturation pulse) for suppressing signals of inflow blood from at least one inflow blood vessel other than a specific inflow blood vessel among the plurality of inflow blood vessels may be applied immediately before the tag-on data acquisition sequence. Similarly, in each of the tag-off sequences of the second pulse sequence group as shown in Fig. 4B, the suppression pulse for suppressing the signals of the inflow blood from the inflow blood vessel(s) other than the specific inflow blood vessel among the plurality of inflow blood vessels may be applied immediately before the tag-off data acquisition sequence.

Both the tag-off data acquisition sequence and the tag-on data acquisition sequence are not limited to a specific type. When the imaging region is a 2D (Two-Dimensional) slice region, the sequence type of both may be a 2D-FSE (Fast Spin Echo), a 2D-EPI, or a 2D-FFE (Fast Field Echo), for example. When the imaging region is a 3D (Three-Dimensional) slab region, the sequence type of both may be a 3D-FSE or 3D-FFE, for example. The pulse sequences shown in Fig. 4A, Fig. 4B, and Fig. 5A to Fig. 5D are called a Flow-Out method because they are an imaging method of depicting outflow of the blood flowing out of the application region of the tag pulse.

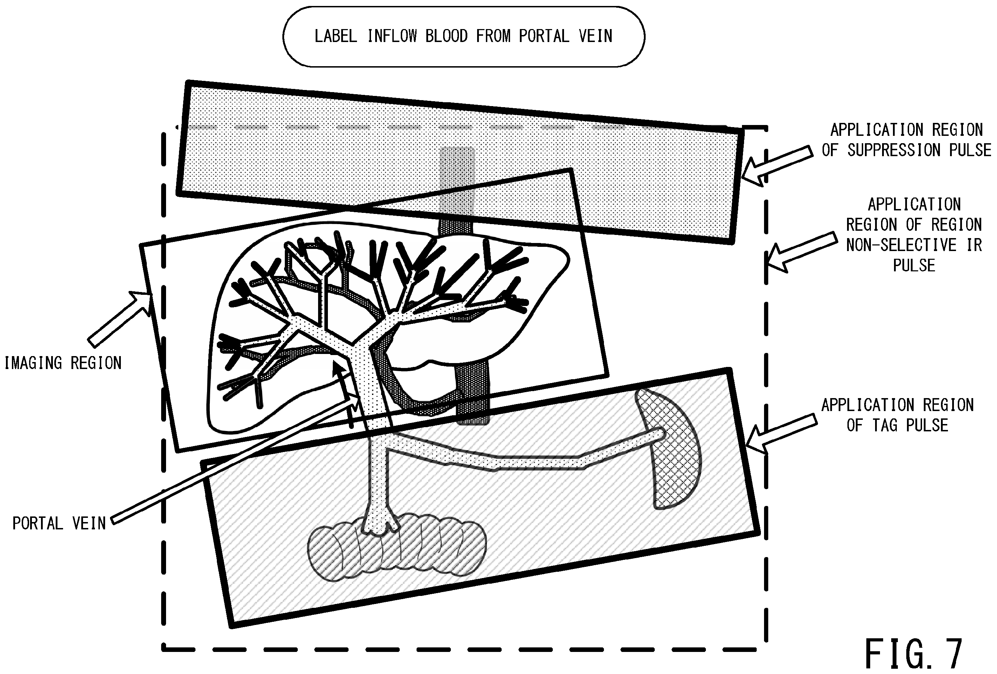

The imaging region and the respective application regions of the region non-selective IR pulse, the region selective IR pulse (i.e., tag pulse), and the suppression pulse will be described below by using Fig. 7, Fig. 10 to Fig. 12, and Fig. 16A to Fig. 17C.

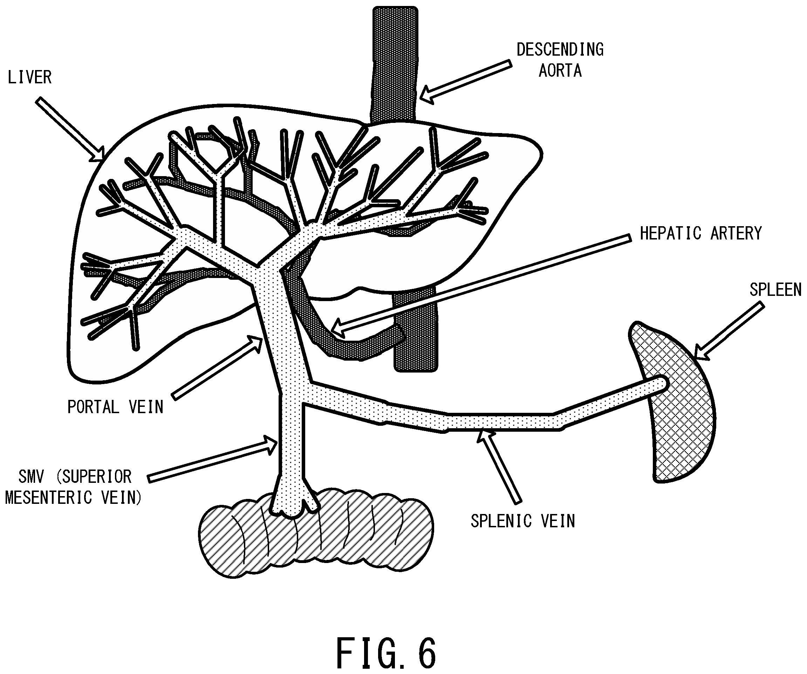

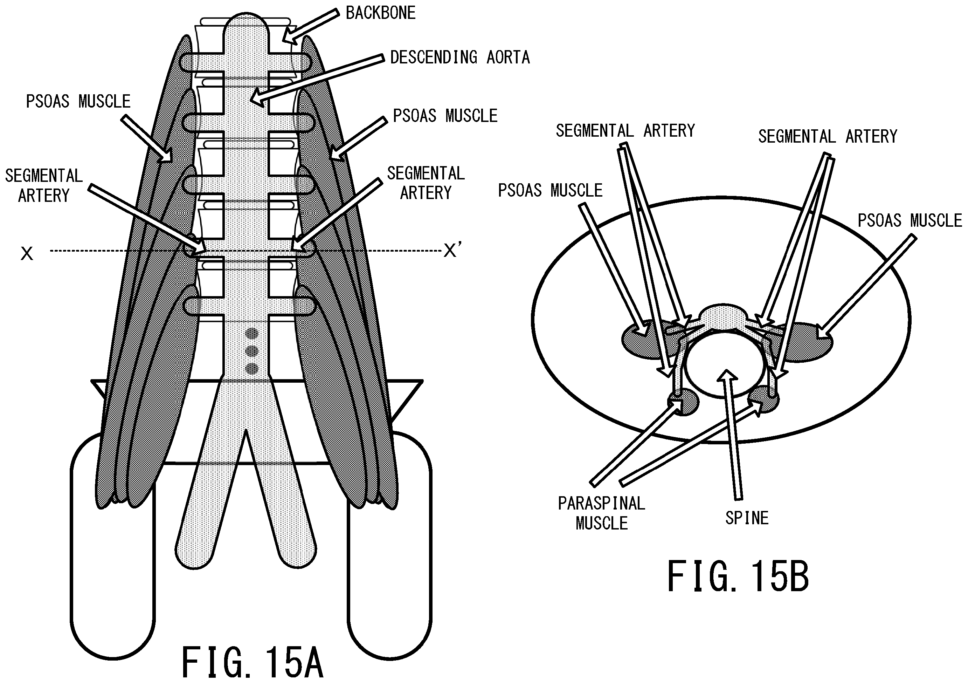

As described above, the main imaging target of the MRI apparatus 1 of the embodiment is a tissue or organ into which blood flows from a plurality of inflow blood vessels. In the following, the operation of the MRI apparatus 1 for the case of imaging the liver as one example of such an organ will be described as a first example by using Fig. 6 to Fig. 14. Further, the operation of the MRI apparatus 1 for the case of imaging the the psoas muscle or the paraspinal muscle as another example of such a tissue will be described as a second example by using Fig. 15A to Fig. 17C.

### (First Example)

Fig. 6 is a schematic diagram illustrating inflow blood vessels with respect to the liver. As is well known, the liver is an organ into which blood flows through two inflow blood vessels that are the portal vein and the hepatic artery. Of these, the portal vein is a vein where blood flowing through the splenic vein from the spleen and blood flowing through the superior mesenteric vein (SMV) from mainly the small intestine join together and flow into the liver.

Regarding the hepatic artery, blood branching off from the descending aorta flows into the hepatic artery and this blood flows into the liver. In this manner, blood flows into the liver from the two inflow blood vessels, i.e., the hepatic artery and the portal vein. Further, the blood from at least two inflow blood vessels, i.e., the splenic vein and the superior mesenteric vein, joins and flows into the portal vein and then flows into the liver.

For such an organ having a plurality of inflow blood vessels, the MRI apparatus 1 of the embodiment enables acquisition of perfusion curves and metrics related to perfusion of the blood for each position in the organ in such a manner that the blood is separated into individual inflow blood vessels.

Fig. 7 is a schematic diagram illustrating an application region of RF pulses such as a tag pulse for labeling blood flowing into the liver from the portal vein, from among these inflow blood vessels. Note that labeling can also be referred to as tagging.

As shown in Fig. 7, the region non-selective pulse is applied not only to the liver but also to the region including all the inflow blood vessels flowing into the liver (the descending aorta, the hepatic artery, the portal vein, the splenic vein, and the superior mesenteric vein). In order to label the blood from the portal vein, the tag pulse is applied to the region including the portal vein. In this case, the tag pulse may be applied to the region including not only the portal vein but also the splenic vein and the superior mesenteric vein.

Although the region non-selective pulse suppresses signals of the blood from the blood vessels other than the portal vein (in this case, the hepatic artery), in order to further suppress signals of the blood from the hepatic artery, a suppression pulse may be applied to the region of the descending aorta on the upstream side of the hepatic artery.

Returning to Fig. 3, in the step ST101, tag-on images and tag-off images of the tissue or organ of the object are reconstructed for each delay time TI, based on the MR signals emitted from the object by applying the above-described pulse sequence group,.

In the subsequent step ST102, the signal intensity ratios (SIR) are calculated as perfusion data from the tag-on images and the tag-off images, and perfusion images are generated for the respective delay times TI.

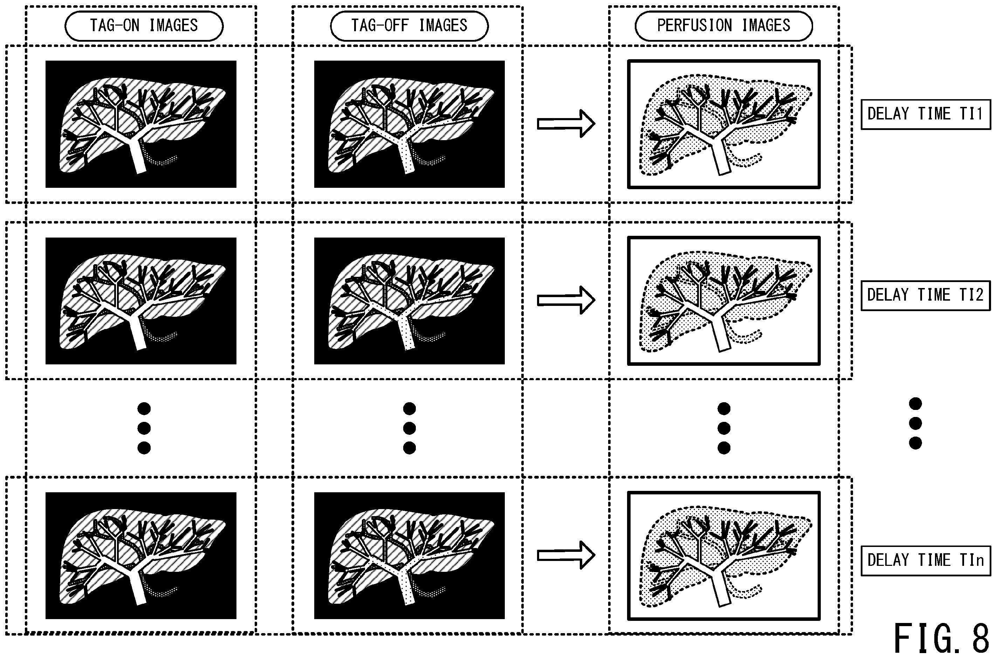

Fig. 8 is a schematic diagram illustrating a concept of the processing of the steps ST101 and ST102. The left row of Fig. 8 illustrates tag-on images that are generated by using the tag-on data acquired under the above-described tag-on sequence. The tag-on images are generated for each delay time TI. The central row of Fig. 8 illustrates tag-off images that are generated by using the tag-off data acquired under the above-described tag-off sequence. The tag-off images are also generated for each delay time TI.

Each signal intensity ratio SIR calculated as the perfusion data in the step ST102 is obtained by dividing the absolute value of the difference between the pixel value of the tag-off image generated from the tag-off data and the pixel value of the tag-on image generated from the tag-on data at each pixel position corresponding to each other by the absolute value of the pixel value of the tag-off image at the shortest delay time TImin among the plurality of delay times TI, for example. The signal intensity ratio (SIR) is calculated by Expression 1 below, for example. $SIR \left(TI\right) = \frac{\left|{S}_{off}\left(TI\right)-{S}_{on}\left(TI\right)\right|}{\left|{S}_{off}\left(TImin\right)\right|}$ Expression 1

In Expression 1, S_{off}(TI) is the pixel value (i.e., signal intensity) of the tag-off image corresponding to the delay time TI, and Sₒₙ(TI) is the pixel value (i.e., signal intensity) of the tag-on image corresponding to the delay time TI. Further, S_{off}(TImin) used for division is the pixel value of the tag-off image at the shortest delay time TImin among the plurality of delay times TI.

Alternatively, as a modification of Expression 1, the signal intensity ratio (SIR) may be determined by dividing the numerator by the pixel value S_{off}(TImax) of the tag-off image at the longest delay time among the plurality of delay times TI, as shown in Expression 2 below. $SIR \left(TI\right) = \frac{\left|{S}_{off}\left(TI\right)-{S}_{on}\left(TI\right)\right|}{\left|{S}_{off}\left(TImax\right)\right|}$ Expression 2

Further alternatively, as a modification of Expression 1, the signal intensity ratio (SIR) may be determined by dividing the numerator by the pixel value Soff(TIpeak) of the tag-off image at which the blood vessel to be observed gives the strongest signal among the plurality of delay times TI, as shown in Expression 3 below. $SIR \left(TI\right) = \frac{\left|{S}_{off}\left(TI\right)-{S}_{on}\left(TI\right)\right|}{\left|{S}_{off}\left(TIpeak\right)\right|}$ Expression 3

Which of Expressions 1, 2, or 3 is used to calculate the signal intensity ratios (SIR) can be selected depending on the target blood vessel. The SIR(TI) values to be calculated by Expression 1, 2, or 3 are the signal intensity ratios corresponding to the delay times TI, and are the perfusion data calculated for each pixel position of the tag-on image and the tag-off image. The image in which the SIR(TI) values are arranged as pixel values at the corresponding pixel positions is called a perfusion image. The right row of Fig. 8 illustrates a perfusion image generated in this manner.

Returning to Fig. 3, in the step ST103, the perfusion curve corresponding to each inflow blood vessel is calculated from the SIR for each delay time (TI) for each pixel or subregion of the tissue or organ.

In the next step ST104, the metrics related to the perfusion are calculated from the perfusion curve.

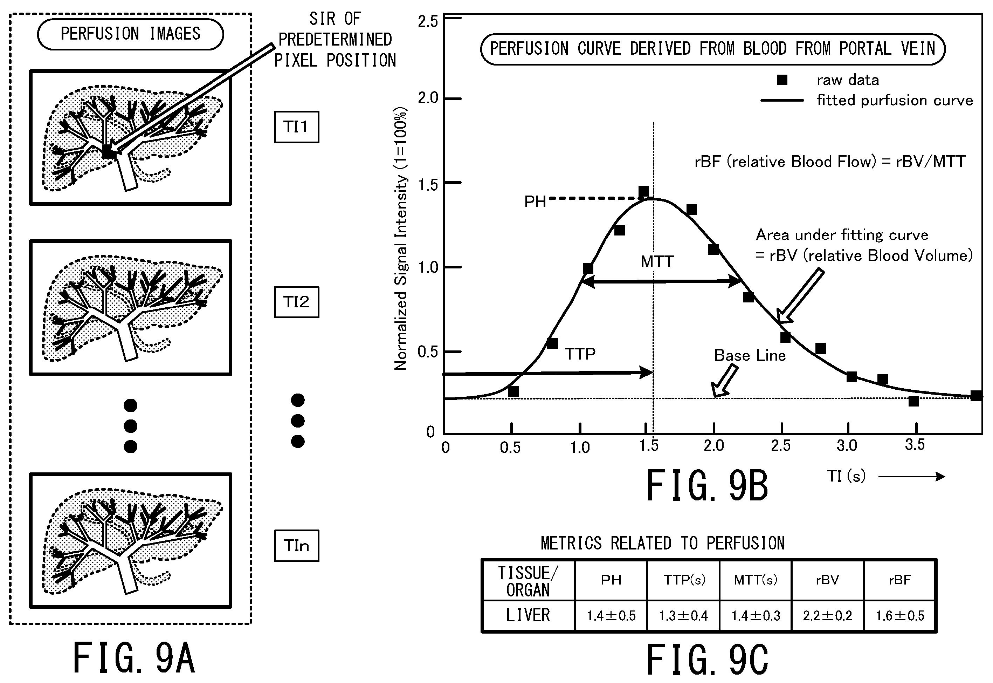

Fig. 9A to Fig. 9C are schematic diagrams illustrating a concept of the processing of the steps ST103 and ST104. Fig. 9A is the same as the right row of Fig. 8, and illustrates the perfusion images for each delay time TI generated in the step ST102. Fig. 9B is a graph illustrating a perfusion curve calculated from the SIR at a specific pixel position in the plurality of perfusion images for each delay time TI. The horizontal axis of the graph indicates the delay time TI, and the vertical axis indicates the SIR.

The perfusion curve can be generated by fitting the SIR corresponding to the plurality of discrete delay times TI, which are the measured values, with a gamma variate function, for example.

Furthermore, metrics such as PH (peak height), MTT (Mean Transition Time), TTP (time to peak), rBV (relative Blood Volume), and rBF (relative Blood Flow) can be calculated as the metrics related to the perfusion from the generated perfusion curve. As shown in Fig. 9C, the metrics related to perfusion can be calculated as the numerical values of respective metrics.

It should be noted that the perfusion curve or the perfusion-related metrics calculated in the steps ST103 and ST104 are related to perfusion of the blood flowing into the liver from at least one specific inflow blood vessel selected from the plurality of inflow blood vessels flowing into the liver. In the above-described case, the MRI apparatus 1 calculates the perfusion curve and metrics of the blood flowing from the portal vein into the liver among the plurality of inflow blood vessels flowing into the liver.

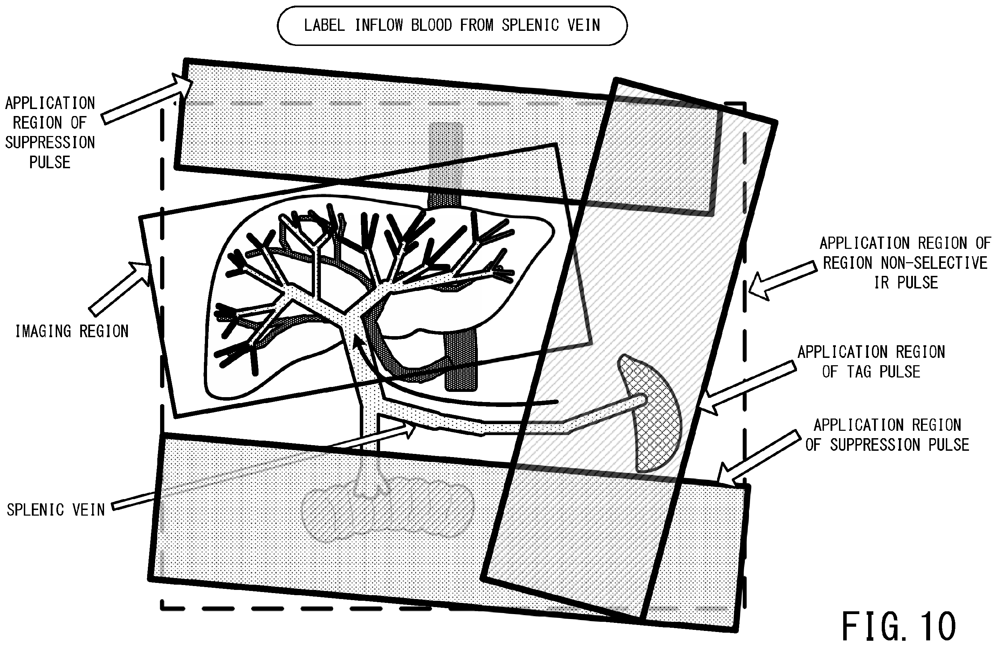

In the MRI apparatus 1 of the embodiment as shown in Fig. 10 to Fig. 12, the perfusion curve and the perfusion-related metrics of the blood flowing into the liver from a desired specific inflow blood vessel among the plurality of inflow blood vessels flowing into the liver can be calculated by changing the application region of the tag pulse and/or the application region of the suppression pulse.

Fig. 10 is a schematic diagram illustrating respective application regions of the suppression pulse and the tag pulse for labeling the blood flowing into the liver from the splenic vein via the portal vein. In this case, the tag pulse is applied to the region that includes the spleen and the splenic vein extending from the spleen toward the portal vein. The suppression pulse is applied to both the region including the superior mesenteric vein and the region including the descending aorta from which the hepatic artery branches.

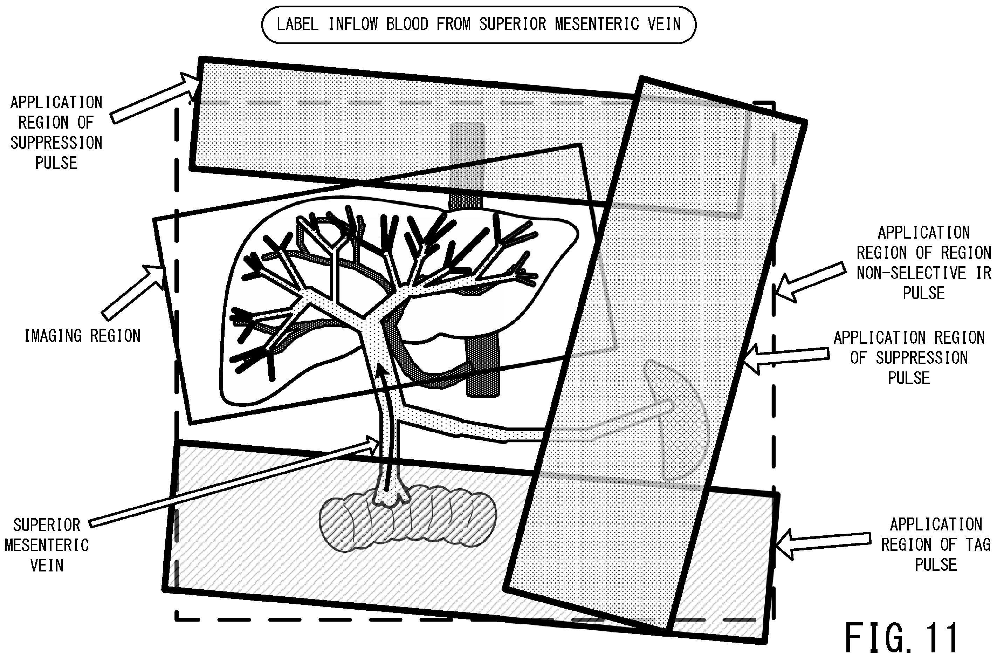

Fig. 11 is a schematic diagram illustrating respective application regions of the suppression pulse and the tag pulse for labeling the blood flowing into the liver from the superior mesenteric vein via the portal vein. In this case, the tag pulse is applied to the region that includes (a) the upper portion of the small intestine and (b) the superior mesenteric vein extending from the upper portion of the small intestine toward the portal vein. The suppression pulse is applied to both the region including the spleen and splenic veins and the region including the descending aorta from which the hepatic artery branches.

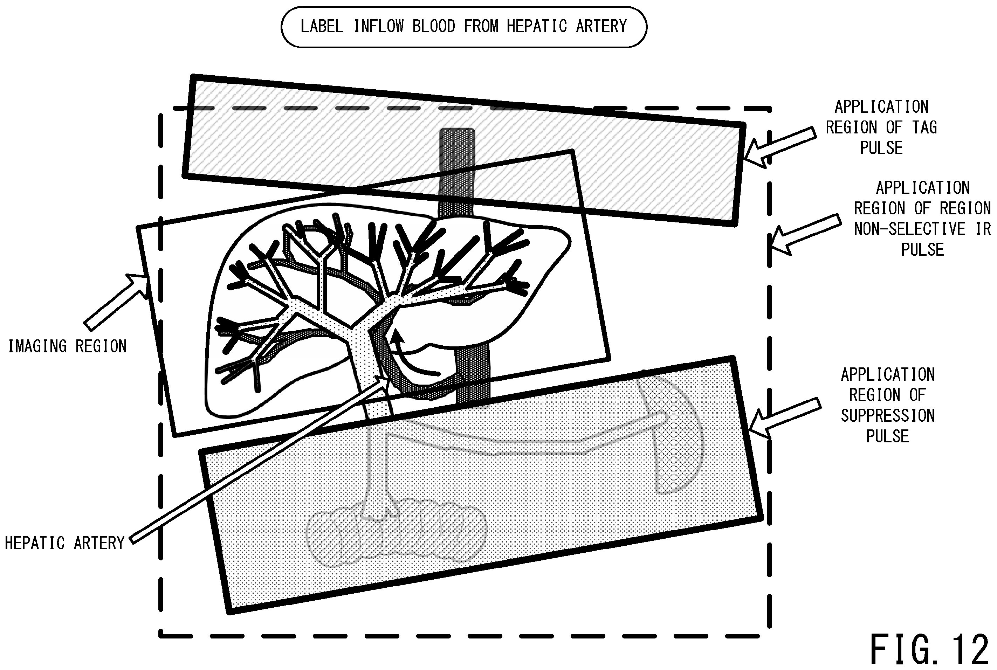

Fig. 12 is a schematic diagram illustrating respective application regions of the suppression pulse and the tag pulse for labeling the blood flowing into the liver via the hepatic artery. In this case, the tag pulse is applied to the region including the descending aorta on the upstream side of the hepatic artery. The suppression pulse is applied to the region that includes the upper portion of the small intestine, the superior mesenteric vein, the spleen, the splenic vein, and the portal vein.

In this manner, the perfusion curves and the metrics related to perfusion can be calculated for each part of the liver (for each pixel or voxel of the hepatic image data) by individually labeling the plurality of inflow blood vessels flowing into the liver.

The processing of the steps ST103 and ST104 is performed by the perfusion-curve calculation function F04 and the perfusion-metric calculation function F05 of the processing circuitry 40. The perfusion-curve calculation function F04 and the perfusion-metric calculation function F05 calculate respective perfusion curves corresponding to the inflow blood vessels of the hepatic artery, the portal vein, the splenic vein, and the superior mesenteric vein, and further calculates the metrics related to perfusion from the perfusion curves for each subregion obtained by dividing the entire liver region into plural subregions or for each location (voxel) of the liver.

The perfusion-curve calculation function F04 and/or the perfusion-metric calculation function F05 may calculate a combined perfusion curve by combining all of the perfusion curves corresponding to the respective inflow vessels of the hepatic artery, the portal vein, the splenic vein, and the superior mesenteric vein, and further calculate at least one perfusion-related metric for the entire liver region from the combined perfusion curve.

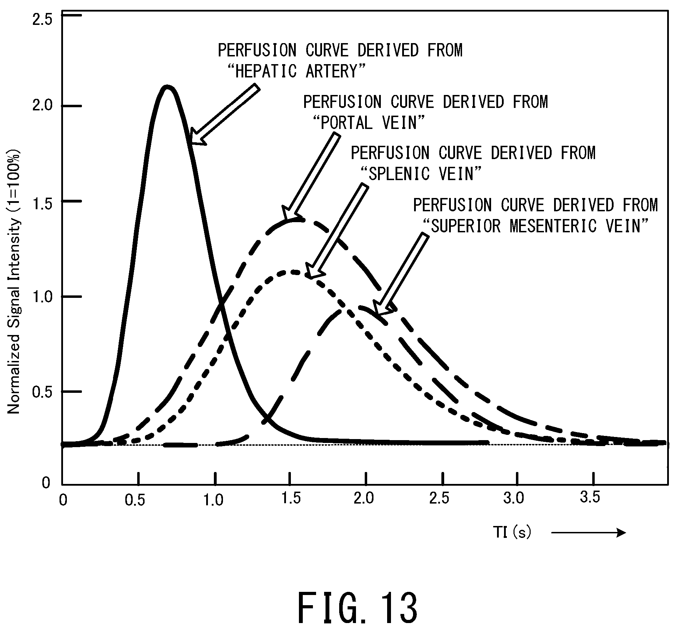

Fig. 13 is a graph illustrating respective perfusion curves derived from the hepatic artery, the portal vein, the splenic vein, and the superior mesenteric vein. As shown in Fig. 13, it is conceivable that the perfusion curves show different shapes depending on each inflow blood vessel. Here, the shape of the perfusion curve changes depending on various parameters such as signal intensities and time delays. Thus, the fact that shapes of perfusion curves are different includes that signal intensities are different and/or time delays are different.

Since the locations of the branching vessels from each inflow blood vessel are different in the liver, it is conceivable that the perfusion curves corresponding to the respective inflow blood vessels show different shapes (including either or both different signal intensities and different time delays) also depending on their locations in the liver.

In the step ST105 of Fig. 3, on the basis of these characteristics of the perfusion curves, the evaluation function F07 of the processing circuitry 40 evaluates the state of the tissue or organ by using at least one of the metrics related to perfusion and the shape of the perfusion curve.

For example, with the use of at least one of the shapes of the perfusion curves corresponding to the calculated respective inflow blood vessels and the metrics calculated from the perfusion curves, the evaluation function F07 can classify subregions of the liver depending on which of the hepatic artery, the portal vein, the splenic vein, and the superior mesenteric vein is the dominant source of inflow blood.

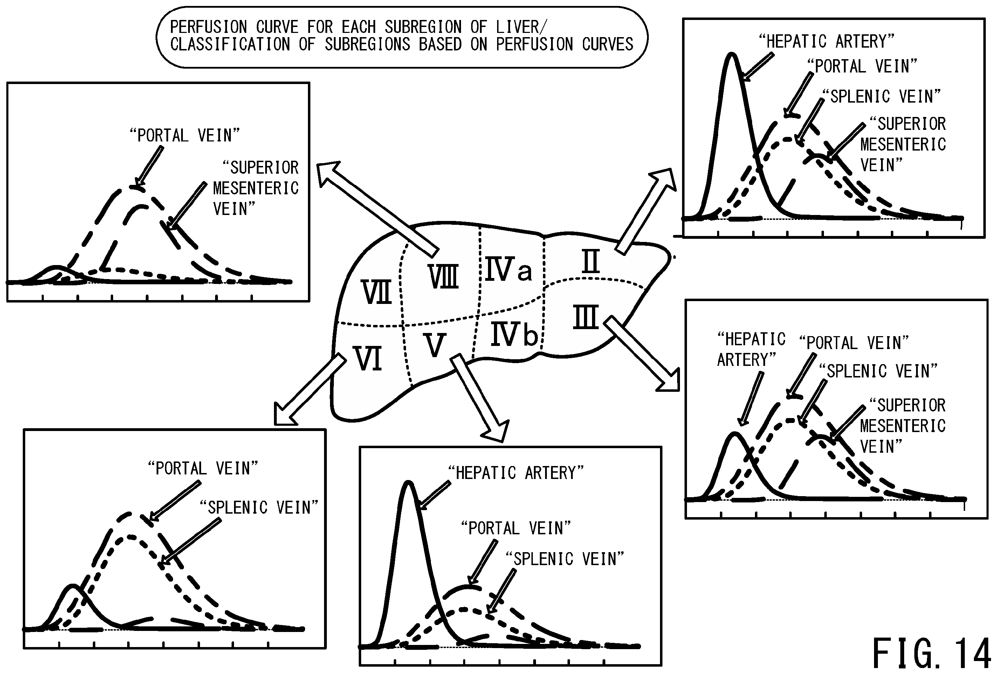

For example, as shown in Fig. 14, the entire liver region can be divided into a plurality of subregions as shown by the region I to the region VIII (region I is on the back side of the sheet of the figure and is not illustrated in Fig. 14), and the entire liver region can be classified for each subregion on the basis of which of the hepatic artery, the portal vein, the splenic vein, and the superior mesenteric vein is the dominant source of inflow blood.

For example, the region II and the region V can be classified as regions where inflow blood from the hepatic artery is dominant. For example, the region VI and the region VIII can be classified as regions where inflow blood from the portal vein is dominant. The classification of the liver regions (i.e., hepatic segmentation) based on which of these blood vessels is the dominant source of inflow blood can be of great help in determining the surgical procedure for tumor removal.

The evaluation function F07 can evaluate the state of the tissue or organ of the object such as a patient by comparing the standard metrics in the diagnostic database 410, which stores perfusion curves and perfusion-related metrics of healthy persons, with perfusion-related metrics calculated from the perfusion curves, which are generated during an examination or diagnosis of the object.

Based on at least one of the shapes of the perfusion curves corresponding to the calculated respective inflow blood vessels and the metrics calculated from the perfusion curves, the evaluation function F07 can also evaluate whether the tumor occurring at each location or each subregion in the liver originates from an inflow blood vessel such as the hepatic artery, the portal vein, the splenic vein, and the superior mesenteric vein or not.

The display control function F08 may cause the display 42 to appropriately display the respective perfusion curves calculated for the plurality of inflow blood vessels, at least one perfusion-related metric, evaluation results acquired by the evaluation function F07, and at least one perfusion image.

### (Second Example)

The operation of the MRI apparatus 1 for the case of imaging the psoas muscle or the paraspinal muscle will be described as the second example by using Fig. 15A to Fig. 17C.

Fig. 15A and Fig. 15B are schematic diagrams illustrating the psoas muscle and the paraspinal muscle in the human body. Fig. 15A and Fig. 15B are intended to illustrate the operation of the MRI apparatus 1 in the case of imaging the psoas muscle or the paraspinal muscle, and are not intended to be anatomically accurate.

As shown in Fig. 15A and Fig. 15B, the psoas muscle is a muscle that connects the lumbar vertebrae at the lower portion of the backbone to the femur and also connects each of the plurality of vertebrae constituting the lumbar vertebrae (i.e., individual bones constituting the segments of the spine) to the femur. The psoas muscle has an important role in adjusting the tilt of the pelvis, straightening the spine, and maintaining a good posture.

The paraspinal muscle is located near the backbone from the neck to the lower back as shown in Fig. 15B, and contributes to the protection and stability of the lumbar vertebrae.

The psoas muscle and the paraspinal muscle are supplied with blood from the segmental arteries, which act as feeding vessels. As shown in Fig. 15A and Fig. 15B, the segmental arteries are a plurality of arteries that branch off from the descending aorta at each vertebra and supply blood to the bone marrow and muscles such as the psoas muscle and the paraspinal muscle.

Thus, the psoas muscle and the paraspinal muscle are tissues into which blood flows from a plurality of inflow blood vessels (i.e., a plurality of segmental arteries).

Application of the above-described pulse sequence group to such a tissue enables calculation of the perfusion curve caused by inflow blood from a specific segmental artery among the plurality of segmental arteries and/or the metrics related to perfusion for such a tissue.

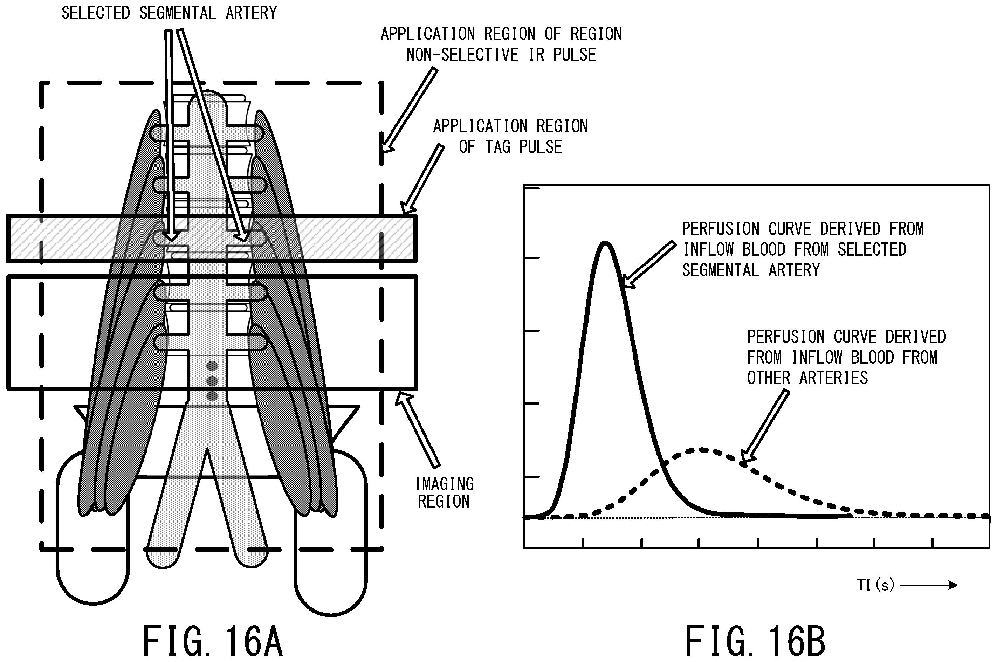

Fig. 16A and Fig. 16B are conceptual diagrams illustrating processing of selecting a specific segmental artery from among a plurality of segmental arteries and calculating the perfusion curve of inflow blood from the selected segmental artery. In this processing as shown in Fig. 16A, the region non-selective IR pulse is applied to the region including the entire psoas muscle in both the tag-on sequence and the tag-off sequence. The tag pulse in the tag-on sequence is applied to the region including only the desired segmental artery in such a manner that only the inflow blood flowing from the desired segmental artery is selected. The imaging region is then set on the downstream side of the selected segmental artery.

Since the imaging region and the respective application regions of the region non-selective IR pulse and the tag pulse are set in this manner, the perfusion curve due to inflow blood from the selected segmental artery and the perfusion curve due to inflow blood from other arteries can be separately calculated as shown in Fig. 16B.

Based on at least one of the shapes of the calculated perfusion curves corresponding to the inflow blood vessels from the respective segmental arteries and the metrics calculated from the perfusion curves obtained under such processing, for example, the evaluation function F07 of the processing circuitry 40 can evaluate presence/absence of an abnormality and which of the plurality of segmental arteries has an abnormality in supply of inflow blood in the case of existence of the abnormality. For example, the evaluation function F07 can evaluate which segmental artery has stopped supply of inflow blood or is reduced in supply of inflow blood.

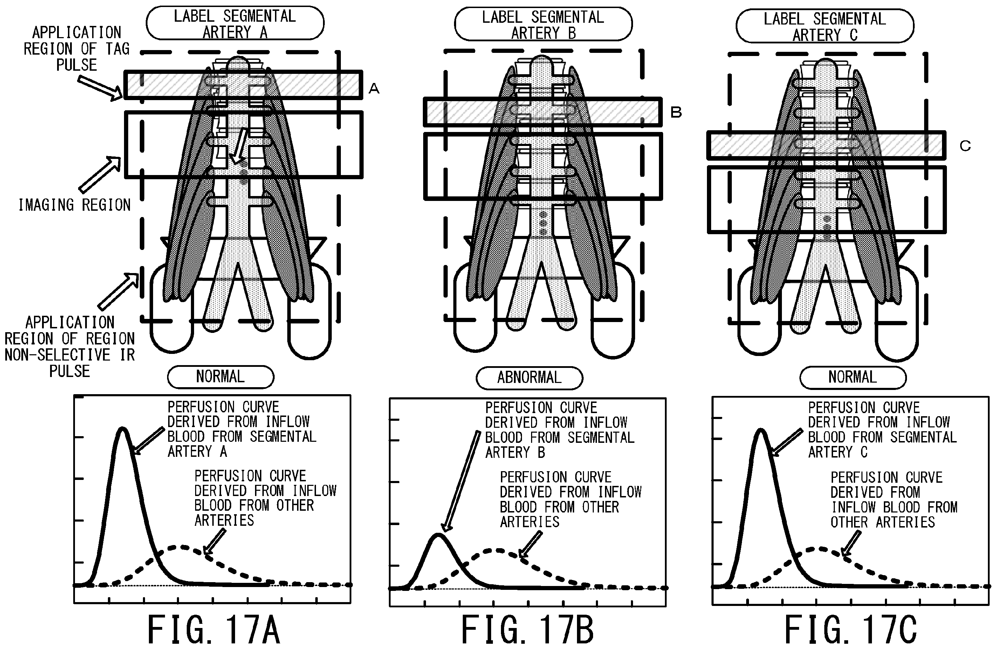

Fig. 17A to Fig. 17C illustrate such an evaluation function. Fig. 17A illustrates a case where the tag pulse is applied to a segmental artery A and the perfusion curve of the inflow blood from the segmental artery A is calculated. Fig. 17B illustrates a case where the tag pulse is applied to a segmental artery B and the perfusion curve of the inflow blood from the segmental artery B is calculated. Fig. 17C illustrates a case where the tag pulse is applied to a segmental artery C and the perfusion curve of the inflow blood from the segmental artery C is calculated.

As can be seen from Fig. 17A to Fig. 17C, the PH (peak height) of the perfusion curve of the inflow blood from the segmental artery B is significantly lower than that of segmental arteries A and C. In such a case, the evaluation function F07 of the processing circuitry 40 can evaluate that some abnormality has occurred in supply of the inflow blood from the segmental artery B.

In addition to the liver, the psoas muscle, and the paraspinal muscle as mentioned above, the brain is also considered to have a plurality of inflow blood vessels. The brain has a plurality of inflow blood vessels including the right and left internal carotid arteries and the right and left vertebral arteries. The MRI apparatus 1 of the embodiment can calculate the perfusion curve and/or perfusion-related metrics caused by the inflow blood from a specific inflow blood vessel among these plurality of inflow blood vessels. Furthermore, even in the case of a tissue or organ excluding the liver, the psoas muscle, the paraspinal muscle, and the brain, the state of the tissue or organ having a plurality of inflow blood vessels can be evaluated for each inflow blood vessel by applying the processing of the above-described embodiment to the tissue or organ.

The plurality of inflow paths into the tissue or organ are not limited to the blood vessels described above. The inflow from the plurality of inflow paths into the tissue or organ is not limited to blood. The plurality of inflow paths may also be body cavities, such as a subarachnoid space, i.e., spaces within the body. The inflow from the plurality of inflow paths into the tissue or organ may be body fluid other than blood, such as cerebrospinal fluid (CSF). In other words, perfusion data and perfusion curves may be attributable not only to inflowing blood but also to inflowing body fluid.

According to the MRI apparatus of the embodiment as described above, even in the case of a tissue or organ having a plurality of inflow paths, the perfusion curve indicative of the behavior of body fluid flowing into the tissue or organ and metrics related to the perfusion can be calculated for each of the plurality of inflow paths, and consequently, the state of the tissue or organ can be evaluated for each inflow path in more detail.

Note that the imaging condition setting function F01 in the description of the embodiment is an example of a setting unit. The reconstruction processing function F02, the SIR calculation function F03, and the perfusion-curve calculation function F04 in the description of the embodiment are examples of a generation unit. The perfusion-metric calculation function F05 in the description of the embodiment is an example of a calculation unit. The evaluation function F07 is an example of an evaluation unit. The display control function F08 is an example of a display control unit.

While certain embodiments have been described, these embodiments have been presented by way of example only, and are not intended to limit the scope of the invention. Indeed, the novel methods and systems described herein may be embodied in a variety of other forms; furthermore, various omissions, substitutions and changes in the form of the methods and systems described herein may be made without departing from the scope of the invention as defined by the appended claims. The accompanying claims are intended to cover such forms or modifications as would fall within the scope of the invention.

## Claims

1. An MRI apparatus comprising:
a setting unit configured to set a pulse sequence group to be applied to a tissue or organ into which body fluid flows from a plurality of inflow paths, the pulse sequence group being provided with RF pulses for labeling body fluid in the plurality of inflow paths,
a generation unit configured to generate perfusion data regarding perfusion of the body fluid flowing into the tissue or organ of the body fluid from each of the plurality of inflow paths based on MR signals that are emitted from an object by application of the pulse sequence group and received with a receiving coil, and
a calculation unit configured to calculate metrics related to the perfusion in the tissue or organ for each of the plurality of inflow paths based on the perfusion data.

2. The MRI apparatus according to claim 1, wherein the inflow path is an inflow blood vessel, and the body fluid is blood.

3. The MRI apparatus according to claim 1 or 2, wherein the pulse sequence group to be set by the setting unit is a pulse sequence group corresponding to an imaging method in which (a) a tag pulse is applied to a predetermined tag region including a specific inflow path among the plurality of inflow paths and then tag-on data are acquired from a predetermined imaging region in the tissue or organ with a plurality of different delay times TI from application of the tag pulse, and (b) tag-off data are acquired from the predetermined imaging region without applying the tag pulse.

4. The MRI apparatus according to claim 3, wherein:
the pulse sequence group to be set by the setting unit includes a first pulse sequence group for acquiring the tag-on data and a second pulse sequence group for acquiring the tag-off data;
the first pulse sequence group is a pulse sequence group in which a tag-on sequence is repeated for the plurality of delay times TI, the tag-on sequence including a first region non-selective IR pulse, a first region selective IR pulse as the tag pulse subsequent thereto, a first acquisition sequence provided after the delay time TI from application of the first region selective IR pulse; and
the second pulse sequence group is a pulse sequence group in which a tag-off sequence including a second region non-selective IR pulse and a second acquisition sequence is repeated for the plurality of delay times TI, the tag-off sequence being a sequence in which an interval between the second region non-selective IR pulse and the second acquisition sequence is set to be same as an interval between the first region non-selective IR pulse and the first acquisition sequence.

5. The MRI apparatus according to claim 4, wherein:
the first pulse sequence group to be set by the setting unit includes a suppression pulse applied immediately before the first acquisition sequence, the suppression pulse being for suppressing a signal of inflow body fluid from at least one inflow path excluding the specific inflow path among the plurality of inflow paths; and
the second pulse sequence group to be set by the setting unit includes the suppression pulse applied immediately before the second acquisition sequence.

6. The MRI apparatus according to claim 3, wherein:
the perfusion data generated by the generation unit are signal intensity ratio (SIR) calculated for respective delay times; and
the signal intensity ratio is obtained by dividing an absolute value of difference between a pixel value of a tag-off image generated from the tag-off data and a pixel value of a tag-on image generated from the tag-on data at each pixel position corresponding to each other by an absolute value of a pixel value of the tag-off image at one of a shortest delay time, a longest delay time, or a delay time corresponding to maximizing signal intensity of a body fluid observed among the plurality of delay times.

7. The MRI apparatus according to any one of claims 1 to 6, wherein the generation unit is further configured to calculate a perfusion curve in the tissue or organ for each of the plurality of inflow paths based on the perfusion data.

8. The MRI apparatus according to claim 7, further comprising a display control unit configured to display at least one of the following perfusion curves:
(i) respective perfusion curves calculated for the plurality of inflow paths; and
(ii) a perfusion curve obtained by combining at least two of the respective perfusion curves calculated for the plurality of inflow paths.

9. The MRI apparatus according to claim 7 or 8, wherein the generation unit is further configured to combine at least two of the respective perfusion curves calculated for the plurality of inflow paths.

10. The MRI apparatus according to any one of claims 7 to 9, wherein the calculation unit is further configured to, by using the calculated perfusion curve, calculate at least one of PH (peak height), MTT (mean transition time), TTP (time to peak), rBV (relative blood fluid volume), and rBF (relative blood flow), as the metrics related to the perfusion for each of the plurality of inflow paths.

11. The MRI apparatus according to any one of claims 7 to 10, further comprising a memory configured to store an evaluation database in which standard metrics related to perfusion curves obtained by measurement on a plurality of healthy persons are accumulated, and
an evaluation unit configured to evaluate a state of the tissue or organ of the object by comparing the metrics calculated from the perfusion curve of the object with the standard metrics of the evaluation database.

12. The MRI apparatus according to any one of claims 3 to 11, wherein
the tissue or organ is a liver, and
the specific inflow path is one blood vessel selected from a group consisting of a hepatic artery, a portal vein, a splenic vein, and a superior mesenteric vein.

13. The MRI apparatus according to any one of claims 3 to 11, wherein:
the tissue or organ is a liver;
the specific inflow path is one blood vessel selected from a group consisting of a hepatic artery, a portal vein, a splenic vein, and a superior mesenteric vein; and
the generation unit is further configured to
divide entirety of the liver into a plurality of subregions and
calculate, by using the perfusion data, perfusion curves corresponding to respective inflow paths of the hepatic artery, the portal vein, the splenic vein, and the superior mesenteric vein for each of the plurality of subregions.

14. The MRI apparatus according to claim 13, further comprising an evaluation unit configured to classify the subregions depending on which of the hepatic artery, the portal vein, the splenic vein, and the superior mesenteric vein is a dominant source of inflow body fluid, by using at least one of shapes of calculated perfusion curves corresponding to respective inflow paths and metrics calculated from the perfusion curves.

15. The MRI apparatus according to claim 13, further comprising an evaluation unit configured to evaluate which inflow path among the hepatic artery, the portal vein, the splenic vein, and the superior mesenteric vein is a source of origin for a tumor occurring in a subregion, by using at least one of shapes of calculated perfusion curves corresponding to respective inflow paths and metrics calculated from the perfusion curves.
